(19) Europäisches Patentamt
European Patent Office
Office européen des brevets

(11) Veröffentlichungsnummer: **0 284 555 A1**

# (12) EUROPÄISCHE PATENTANMELDUNG

(21) Anmeldenummer: **88810141.7**

(22) Anmeldetag: **07.03.88**

(51) Int. Cl.4: **C 07 D 263/26**
C 07 C 109/04, C 07 C 133/02

(30) Priorität: **12.03.87 CH 923/87**

(43) Veröffentlichungstag der Anmeldung:
**28.09.88 Patentblatt 88/39**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Eckhardt, Wolfgang, Dr.**
**Breslauerstrasse 14**
**D-7850 Lörrach (DE)**

**Süess, Hans, Dr.**
**Burgunderweg 5**
**CH-4313 Möhlin (CH)**

(54) **Verfahren zur Herstellung von N-Acyl-N-(oxazolidin-2-on-3-yl)-2,6-dialkyl-3-chloranilinen.**

(57) Verfahren zur Herstellung von N-Acyl-N-(oxazolidin-2-on-3-yl)-2,6-dialkyl-3-chloranilinen der Formel I

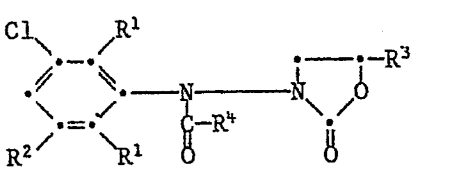

(I)

worin
$R^1$ Methyl oder Aethyl,
$R^2$ Wasserstoff oder Chlor,
$R^3$ Wasserstoff oder Methyl und
$R^4$ $C_1$-$C_3$-Alkoxymethyl, Halogenmethyl oder Tetrahydrofuryl darstellt, indem man ein 2,6-Dialkylanilin der Formel II

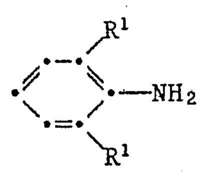

(II)

durch Reaktion mit einem Alkalinitrit diazotiert, das entstandene 2,6-Dialkylphenyldiazoniumsalz mit einem Reduktionsmittel zum 2,6-Dialkylphenylhydrazin der Formel III

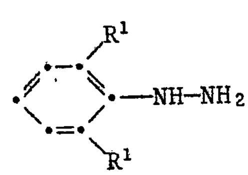

(III)

reduziert, dieses durch Reaktion mit einem Halogenameisensäure-2-halogenäthylester der Formel IV

EP 0 284 555 A1

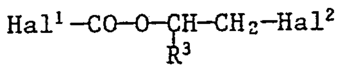

(IV)

worin $Hal^1$ und $Hal^2$ unabhängig voneinander für Chlor oder Brom stehen, in eine Verbindung der Formel V

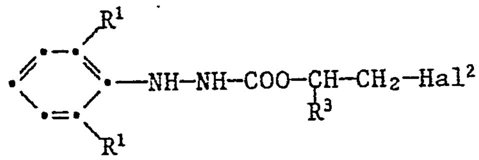

(V)

überführt, dieses Zwischenprodukt entweder zuerst mit einem Acylhalogenid der Formel VI

$Hal^3$-CO-$R^4$ (VI)

worin $Hal^3$ für Chlor oder Brom steht, umsetzt und anschliessend durch Behandlung mit einem säurebindenden Mittel intramolekular cyclisiert; oder zuerst durch Behandlung mit einem säurebindenden Mittel intramolekular cyclisiert und anschliessend mit einem Acylhalogenid der Formel V umsetzt; und das entstehende Zwischenprodukt der Formel VII

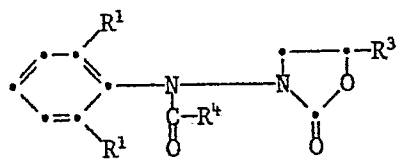

(VII)

durch Einwirkung von Chlor in 3-Stellung einfach oder in 3- und 5-Stellung zweifach chloriert.

**Beschreibung**

Verfahren zur Herstellung von N-Acyl-N-(oxazolidin-2-on-3-yl)-2,6-dialkyl-3-chloranilinen

Die vorliegende Erfindung betrifft ein Verfahren zur Herstellung von N-Acyl-N-(oxazolidin-2-on-3-yl)-2,6-dialkyl-3-chloranilinen der Formel I

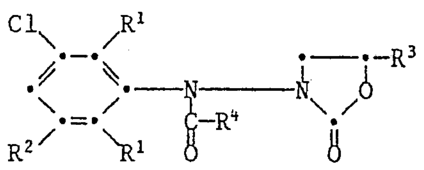

(I)

worin
$R^1$ Methyl oder Aethyl,
$R^2$ Wasserstoff oder Chlor,
$R^3$ Wasserstoff oder Methyl und
$R^4$ $C_1$-$C_3$-Alkoxymethyl, Halogenmethyl oder Tetrahydrofuryl darstellt.

Die Verbindungen der Formel I besitzen fungizide Wirkung. Verbindungen dieser Art sind beispielsweise in der publizierten Britischen Patentanmeldung GB-A-2 058 071, der publizierten Europäischen Patentanmeldung EP-A-30923 und der Deutschen Patentanmeldung DE-OS-3 030 026 beschrieben.

Aus der publizierten Britischen Patentanmeldung GB-A-2 058 071 und der DE-OS-3 030 026 ist es bekannt, die Verbindungen der Formel I aus den zugrundeliegenden 2,6-Dialkyl-3-chloranilinen durch Ueberführung in entsprechende (2,6-Dialkyl-3-chlorphenyl)-hydrazine, aufeinanderfolgende Acylierung an beiden Stickstoffatomen mit 2-Halogenäthylhalogenameisensäureester und einem Carbonsäurehalogenid und intramolekulare Ringschlussreaktion herzustellen. Dieses Verfahren ist insofern nachteilig, als man die einzusetzenden 2,6-Dialkyl-3-chloraniline zuerst nach einem aufwendigen Verfahren synthetisieren muss.

Beispielsweise ist bekannt, 3-Chlor-2,6-dimethylacetanilid durch Chlorierung von 2,6-Dimethylacetanilid in einer Ausbeute von 80 % der Theorie herzustellen (vgl. Synthesis, 1971, Seite 467). Unter Verwendung dieser Methode sind die N-Acyl-N-(oxazolidin-2-on-3-yl)-2,6-dialkyl-3-chloraniline der Formel I ausgehend von einem entsprechenden 2,6-Dialkylanilin durch Acetylierung, Chlorierung des 2,6-Dialkylacetanilids, Hydrolyse des gebildeten 3-Chlor-2,6-dialkylacetanilid zum 3-Chlor-2,6-dialkylanilin, dessen Umsetzung ins Hydrazin, doppelte Acylierung an den beiden Stickstoffatomen und anschliessende intramolekulare Cyclisierung nach folgendem Schema zugänglich:

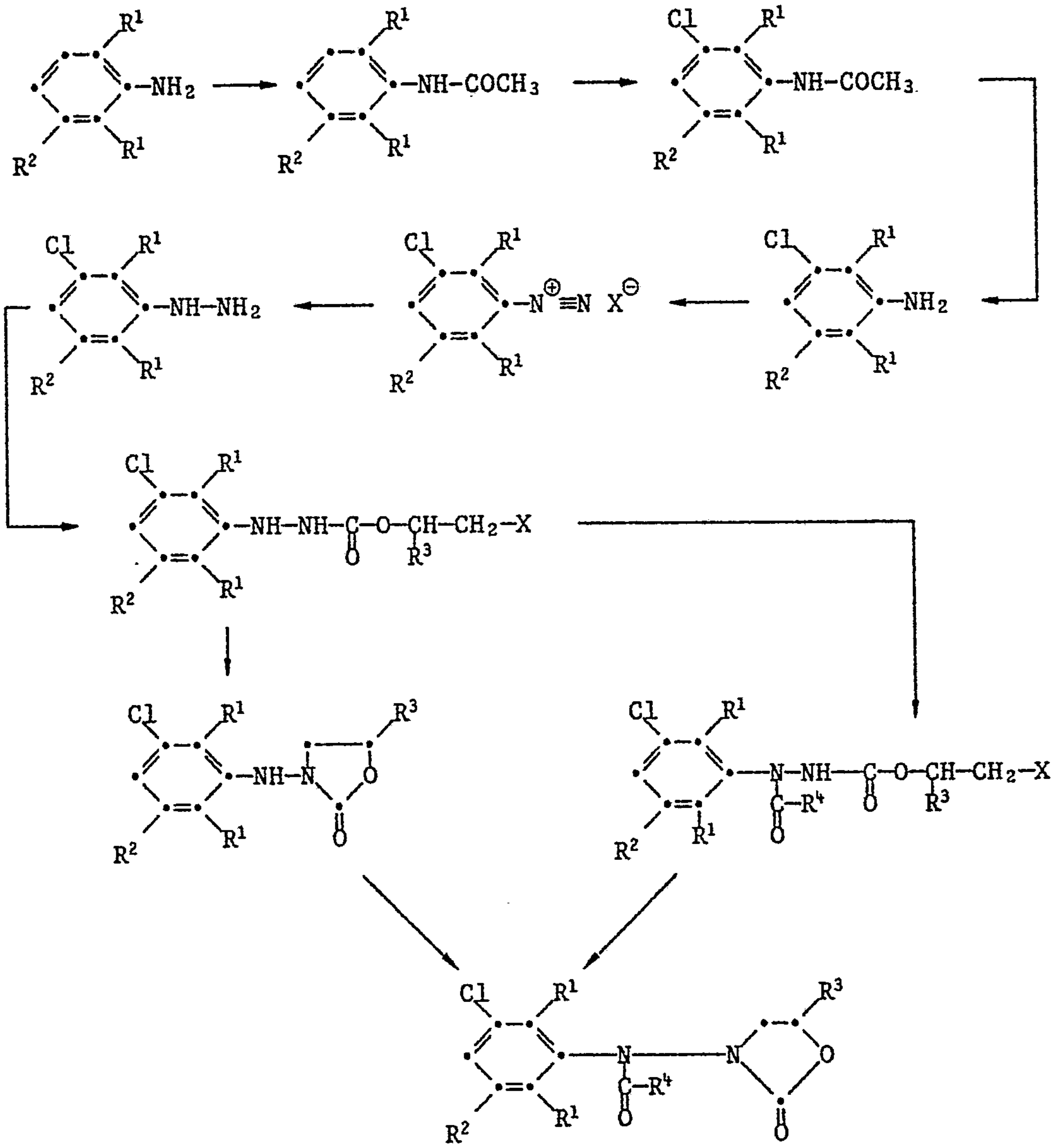

Die Methode ist wegen der grossen Zahl der benötigen Reaktionsstufen umständlich und vermag hinsichtlich der erzielbaren Ausbeuten nicht zu befriedigen.

Es ist daher das Ziel der vorliegenden Erfindung, ein Verfahren bereitzustellen, das die Herstellung der N-Acyl-N-(oxazolidin-2-on-3-yl)-2,6-dialkyl-3-chloraniline der Formel I in einfacher Weise und in guter Ausbeute ermöglicht.

Es wurde gefunden, dass dieses Ziel in vorteilhafter Weise erreicht werden kann, wenn man von einem entsprechenden 2,6-Dialkylanilin ausgeht, das entsprechende Phenylhydrazin daraus herstellt, dieses durch aufeinanderfolgende Acylierung an beiden Stickstoffatomen und intramolekulare Cyclisierung in ein entsprechendes N-Acyl-N-(oxazolidin-2-on-3-yl)-2,6-dialkylanilin überführt und dieses dann durch Einwirkung von Chlor in ein N-Acyl-N-(oxazolidin-2-on-3-yl)-2,6-dialkyl-3-chloranilin der Formel I umsetzt.

Gemäss vorliegender Erfindung wird daher vorgeschlagen, die N-Acyl-N-(oxazolidin-2-on-3-yl)-2,6-dialkyl-3-chloraniline der Formel I in der Weise herzustellen, dass man ein 2,6-Dialkylanilin der Formel II

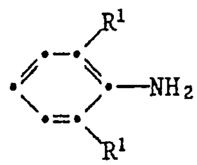

(II)

worin $R^1$ die unter Formel I gegebene Bedeutung hat, durch Reaktion mit einem Alkalinitrit diazotiert, das entstandene 2,6-Dialkylphenyldiazoniumsalz mit einem Reduktionsmittel zum 2,6-Dialkylphenylhydrazin der Formel III

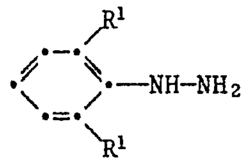

(III)

worin $R^1$ die unter Formel I gegebene Bedeutung hat, reduziert, dieses durch Reaktion mit einem Halogenameisensäure-2-halogenäthylester der Formel IV

$Hal^1-CO-O-\underset{R^3}{CH}-CH_2-Hal^2$ (IV)

worin $R^3$ die unter Formel I gegebene Bedeutung hat und $Hal^1$ und $Hal^2$ unabhängig voneinander für Chlor oder Brom stehen, in eine Verbindung der Formel V

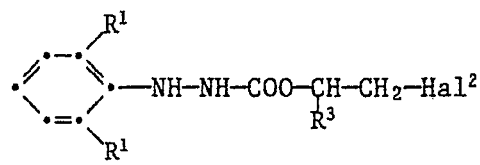

(V)

worin $R^1$ und $R^3$ die unter Formel I gegebene Bedeutung haben und $Hal^2$ für Chlor oder Brom steht, überführt, dieses Zwischenprodukt entweder zuerst mit einem Acylhalogenid der Formel VI

$Hal^3$-CO-$R^4$ (VI)

worin $R^4$ die unter Formel I gegebene Bedeutung hat und $Hal^3$ für Chlor oder Brom steht, umsetzt und anschliessend durch Behandlung mit einem säurebindenden Mittel intramolekular cyclisiert; oder zuerst durch Behandlung mit einem säurebindenden Mittel intramolekular cyclisiert und anschliessend mit einem Acylhalogenid der Formel V umsetzt; und das entstehende Zwischenprodukt der Formel VII

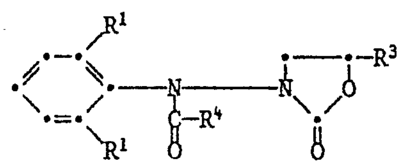

(VII)

worin $R^1$, $R^3$ und $R_4$ die unter Formel I gegebene Bedeutung haben, durch Einwirkung von Chlor in 3-Stellung einfach oder in 3- und 5-Stellung zweifach chloriert.

Geeignete 2,6-Dialkylaniline der Formel II sind 2,6-Dimethylanilin und 2,6-Diäthylanilin. Besonders geeignet ist 2,6-Dimethylanilin.

Als übliche Diazotierungsmittel werden Natriumnitrit und Kaliumnitrit verwendet. Die damit hergestellten Diazoniumsalze werden mit einem Reduktionsmittel zum 2,6-Dialkylphenylhydrazin der Formel III reduziert. In Frage kommen als Reduktionsmittel sowohl Katalysator/Wasserstoff-Systeme wie Platin/$H_2$; Palladium/$H_2$; Platin oxid/$H_2$; als auch Metalle wie Nickel, Zink und Zinn oder Salze dieser Metalle. Bei der Durchführung der Diazotierungsreaktion in Wasser hat es sich als besonders vorteilhaft erwiesen, die Reduktion im gleichen Reaktionsmedium wie die Diazotierung und in homogener Phase vorzunehmen. Zu diesem Zweck werden mit Vorteil wasserlösliche Salze als Reduktionsmittel eingesetzt. Als besonders geeignet hierzu hat sich

Zinn(II)chlorid erwiesen.

Geeignete Halogenameisensäure-2-halogenäthylester der Formel IV sind Chlorameisensäure-2-chloräthylester, Chlorameisensäure-2-bromäthylester oder Bromameisensäure-2-bromäthylester. Bevorzugt ist Chlorameisensäure-2-bromäthylester.

Die Halogenäthoxycarbonylierung des Hydrazins der Formel III mit einem Halogenameisensäure-halogenäthylester der Formel IV wird vorteilhafterweise in einem inerten Lösungsmittel durchgeführt. Die Temperatur des Reaktionsgemisches wird dabei zwischen 0°C und +30°C gehalten, vorzugsweise zwischen +20°C und +30°C. Als inerte Lösungsmittel eignen sich besonders Benzol, Toluol, Xylol, Chlorbenzol, o-Dichlorbenzol, N,N-Dimethylformamid oder N,N-Dimethylacetamid, insbesondere aber Toluol und Xylol.

In bevorzugten Ausführungsformen des erfindungsgemässen Verfahrens wird das 2,6-Dialkylphenylhydrazin der Formel III mit einem Halogenameisensäure-halogenäthylester der Formel IV bei einer Temperatur zwischen +20°C und +30°C in Toluol oder Xylol umgesetzt.

Geeignete Acylierungsmittel der Formel VI sind Chloride und Bromide der Chloressigsäure, Alkoxyessigsäure und Tetrahydrofuran-2-carbonsäure, sowie die Anhydride dieser Säuren, wobei unter Alkoxyessigsäuren insbesondere solche mit einem $C_1$-$C_3$-Alkoxyrest, wie Methoxy, Aethoxy, Propoxy, und Isopropoxy zu verstehen sind. Bevorzugte Acylierungsmittel sind Methoxyacetylchlorid, Chloracetylchlorid und Tetrahydrofuran-2-carbonsäurechlorid. Besonders bevorzugt ist Methoxyacetylchlorid.

Zur Ueberführung des N-2,6-Dialkyl-phenyl-N′-halogenäthoxycarbonylhydrazins der Formel V in das Zwischenprodukt der Formel VII sind zwei Reaktionsschritte notwendig, nämlich eine intramolekulare Cyclisierungsreaktion der N′-ständigen Halogenäthoxycarbonylgruppe mit dem sie tragenden Stickstoffatom, sowie eine Acylierung des Stickstoffatoms welches die 2,6-Dialkylphenylgruppe trägt. Diese beiden Reaktionsschritte können in beliebiger Reihenfolge nacheinander ausgeführt werden. So kann entweder die Verbindung der Formel V zuerst acyliert und danach intramolekular cyclisiert oder alternativ zuerst intramolekular cyclisiert und anschliessend acyliert werden.

Die Umsetzung eines N-2,6-Dialkylphenyl-N′-halogenäthoxycarbonylhydrazins der Formel V oder des zuvor intramolekular cyclisierten N-(Oxazolidin-2-on-3-yl)-2,6-dialkyl-anilins mit einem Acylierungsmittel der Formel V wird vorteilhaft in einem inerten Lösungsmittel in Anwesenheit oder Abwesenheit eines säurebindenden Mittels durchgeführt. Als inerte Lösungsmittel kommen insbesondere mit Wasser nicht mischbare Lösungsmittel, wie aliphatische und aromatische Kohlenwasserstoffe und Halogenkohlenwasserstoffe in Betracht. Geeignete Lösungsmittel sind beispielsweise Hexan, Benzol, Toluol, Xylol, Chlorbenzol, Methylenchlorid, Chloroform, Kohlenstofftetrachlorid und Aethylenchlorid. Bevorzugte Lösungsmittel sind Toluol und Xylol.

Als säurebindende Mittel, in deren Gegenwart die Umsetzung eines N-2,6-Dialkylphenyl-N′-halogenäthoxycarbonyl-hydrazins der Formel V oder des zuvor intramolekular cyclisierten N-(Oxazolidin-2-on-3-yl)-2,6-dialkyl-anilins mit einem Acylierungsmittel der Formel V durchgeführt werden kann, kommen anorganische und organische Basen, wie Alkali- und Erdalkalimetallhydroxide, -carbonate und -hydrogencarbonate, Triäthylamin und Pyridin in Betracht. Vorzugsweise wird die Umsetzung mit einem Acylierungsmittel der Formel VI in Abwesenheit einer Base in Toluol oder Xylol und bei erhöhter Temperatur durchgeführt.

Die Reaktionstemperatur wird vorzugsweise zwischen +40°C und der Siedetemperatur der Reaktionsmischung gehalten.

Die intramolekulare Cyclierung des N-2,6-Dialkylphenyl-N′-halogenäthoxycarbonyl-hydrazins wird vorzugsweise in einem inerten Lösungsmittel in Gegenwart eines säurebindenden Mittels durchgeführt. Die Reaktionstemperatur kann in breiten Grenzen variiert werden, liegt aber im allgemeinen zwischen 0°C und +150°C, vorzugsweise zwischen 0°C und +100°C. Als inertes Lösungsmittel verwendet man mit Vorteil wasserfreie aliphatische oder aromatische Kohlenwasserstoffe oder vorzugsweise Alkohole wie Methanol, Aethanol, Propanol oder Isopropanol oder Gemische solcher Lösungsmittel. Als säurebindende Mittel kommen in erster Linie starke Basen in Betracht wie zum Beispiel Alkali- oder Erdalkalimetallhydroxide, -carbonate-hydrogencarbonate, Triäthylamin oder Pyridin. Bevorzugt sind Natriumhydroxid und Kaliumhydroxid.

Im erfindungsgemässen Verfahren wird vorzugsweise die Verbindung der Formel V zuerst acyliert und anschliessend intramolekular cyclisiert.

In den bevorzugten Ausführungsformen wird die Acylierung des Hydrazin der Formel V mit einem Acylierungsmittel der Formel VI in einem inerten Lösungsmittel ausgeführt. Vorzugsweise werden diese Lösungsmittel aus der Reihe Hexan, Benzol, Toluol, Xylol, Chlorbenzol, Methylenchlorid, Chloroform, Kohlenstofftetrachlorid oder Aethylenchlorid ausgewählt, insbesondere aber aus Toluol oder Xylol. Die Reaktionstemperatur wird mit Vorteil zwischen +40°C und der Siedetemperatur der Reaktionsmischung gehalten. Ganz besonders bevorzugt ist die Durchführung dieser Acylierungsreaktion in Toluol oder Xylol bei einer Temperatur zwischen +40°C und der Siedetemperatur der Mischung. Die anschliessende Cyclisierung zum Zwischenprodukt der Formel VII führt man vorzugsweise in einem inerten Lösungsmittel in Gegenwart eines säurebindenden Mittels durch. Die Lösungsmittel werden vorzugsweise aus der Reihe der niederen aliphatischen Alkohole ausgewählt. Insbesondere können Methanol und Aethanol in Frage. Als säurebindende Mittel können Alkali- und Erdalkalimetallhydroxide, -carbonate, -hydrogencarbonate, Triäthylamin oder Pyridin eingesetzt werden. Die Reaktionstemperatur wird vorzugsweise im Bereich zwischen 0°C und +100°C gehalten. Die Cyclisierungsstufe wird in der bevorzugten Variante bei einer Temperatur zwischen 0°C und +100°C in Methanol oder Aethanol in Gegenwart von Natrium- oder Kaliumhydroxid ausgeführt.

Im bevorzugten erfindungsgemässen Verfahren wird das Zwischenprodukt der Formel V in das Folgeprodukt der Formel VII überführt, indem man die Verbindung der Formel V in Toluol oder Xylol bei einer Temperatur zwischen +40°C und dem Siedepunkt der Reaktionsmischung mit einem Acylierungsreagenz der Formel VI umsetzt und das erhaltene Zwischenprodukt bei einer Temperatur zwischen 0°C und +100°C in Methanol oder Aethanol in Gegenwart von Natrium- oder Kaliumhydroxid cyclisiert.

Die einfache oder zweifache Chlorierung eines N-Acyl-N-(oxazolidin-2-on-3-yl)-2,6-dialkylanilins der Formel VII in 3-Stellung, beziehungsweise in 3- und in 5-Stellung wird ebenfalls vorteilhaft in einem inerten Lösungsmittel vorgenommen. Als Lösungsmittel sind insbesondere niedere aliphatische Carbonsäuren, wie Ameisensäure und Essigsäure geeignet. Ferner können chlorierte aromatische und aliphatische Kohlenwasserstoffe, wie Chlorbenzol, Methylenchlorid, Chloroform, Kohlenstofftetrachlorid und Aethylenchlorid verwendet werden. Die als Lösungsmittel verwendbaren Carbonsäuren können bis zu 60 Gew.% Wasser enthalten. Ein bevorzugtes Lösungsmittel, in dem die Mono-Chlorierung oder die zweifache Chlorierung eines N-Acyl-N-(oxazolidin-2-on-3-yl)-2,6-dialkylanilins der Formel VII durchgeführt werden kann, ist Ameisensäure mit einem Wassergehalt von bis zu 40 Gew.%.

Die Mono-Chlorierung wird vorteilhaft bei Temperaturen von +20°C bis +30°C durchgeführt. Die Mono-Chlorierung kann auch bei tieferen Temperaturen durchgeführt werden. Bei Temperaturen über +40°C werden vorwiegend zweifach chlorierte Produkte gebildet. Bei Temperaturen unter +20°C besteht die Gefahr, dass die Reaktion nicht mehr sofort beim Beginn des Einleitens von Chlor einsetzt, sondern erst in Gang kommt, wenn sich bereits eine relativ hohe Konzentration an Chlor aufgebaut hat. Diese verspätet einsetzende Reaktion verläuft dann sehr heftig und es ist schwierig, die Temperatur des Reaktionsgemisches zu kontrollieren. In diesem Fall muss auch mit der Bildung von uneinheitlich chlorierten Produkten gerechnet werden. Bei Einhaltung der angegebenen Temperaturen lassen sich je nach Wahl des Bereichs zwischen +20°C und +30°C die Mono-Chlorprodukte der Formel I, worin $R^2$ Wasserstoff bedeutet, oder zwischen +40°C und +70°C die zweifach chlorierten Produkte der Formel I, worin $R^2$ Chlor bedeutet, gezielt herstellen.

Es ist ferner vorteilhaft, die beiden Chlorierungsvarianten in Gegenwart von Lewis-Säuren, wie Aluminiumchlorid, Eisen(III)chlorid, Bortrifluorid, und Titantetrachlorid durchzuführen. Eine bevorzugte Lewis-Säure ist Eisen(III)chlorid. Die Lewis-Säuren werden in beiden Fällen in einer Menge von 1-5 Gew.%, vorzugsweise 1,5-2,5 Gew.% bezogen auf das zu chlorierende N-Acyl-N-(oxazolidin-2-on-3-yl)-2,6-dialkylanilin der Formel VII eingesetzt. Die Lewis-Säuren habe an sich keinen wesentlichen Einfluss auf die Chlorierung, sie bewirken jedoch eine starke Erhöhung der Löslichkeit der zu chlorierenden N-Acyl-N-(oxazolidin-2-on-3-yl)-2,6-dialkylaniline der Formel VII in wässriger Ameisensäure oder wässriger Essigsäure. Daher empfiehlt jeweils sich der Zusatz von Lewis-Säuren insbesondere bei Verwendung von Ameisensäure oder Essigsäure als Lösungsmittel, um eine höhere Volumenausbeute zu erreichen. Die Chlorierung eines N-Acyl-N-(oxazolidin-2-on-3-yl)-2,6-dialkylanilins der Formel VII wird daher vorzugsweise zwischen +20°C und +30°C in Ameisensäure mit einem Wassergehalt von bis zu 40 Gew.% in Gegenwart von 1,5-2,5 Gew.% Eisen(III)chlorid bezogen auf eingesetztes N-Acyl-N-(oxazolidin-2-on-3-yl)-2,6-dialkylanilin der Formel VII durchgeführt.

Die Chlorierungen werden in der Regel bei Normaldruck durchgeführt. Bei Verwendung von Ameisensäure oder Essigsäure als Lösungsmittel kann auch unter leichtem Ueberdruck gearbeitet werden, damit bei Verwendung dieser Lösungsmittel kein Gas aus dem Reaktionsgemisch entweicht.

Je nach gewünschten Produkt der Formel I kann durch Wahl der Reaktionstemperatur die Chlorierungsreaktion zum Mono-chlorierten Produkt oder zum zweifach chlorierten Produkt gesteuert werden.

So erhält man in der bevorzugten Ausführungsform die Verbindungen der Formel I, worin $R^2$ für Wasserstoff steht, indem von ein N-Acyl-N-(oxazolidin)-2-on-3-yl)-2,6-dialkylanilin der Formel VII bei +20°C bis +30°C in Ameisensäure mit einem Wassergehalt von bis zu 40 Gew.% in Gegenwart von 1,5-2,5 Gew.% Eisen(III)chlorid bezogen auf eingesetztes N-Acyl-N-(oxazolidin-2-on-3-yl)-2,6-dialkylanilin der Formel VII mit Chlorgas behandelt; und die Verbindungen der Formel I, worin $R^2$ für Chlor steht, indem man N-Acyl-N-(oxazolidin-2-on-3-yl)-2,6-dialkylanilin der Formel VII bei +40°C bis +70°C in Ameisensäure mit einem Wassergehalt von bis zu 40 Gew.% in Gegenwart von 1,5-2,5 Gew.% Eisen(III)chlorid bezogen auf eingesetztes N-Acyl-N-(oxazolidin-2-on-3-yl)-2,6-dialkylanilin der Formel VII mit Chlorgas behandelt.

Die durch das erfindungsgemässe Verfahren leicht zugänglichen Verbindungen der Formel I, worin $R^2$ für Chlor steht, sind neu. Sie besitzen wie ihre monochlorierten, bekannten Mono-Chlor-Analoga herbizide Eigenschaften.

In der besonders bevorzugten Ausführungsform des erfindungsgemässen Verfahrens erhält man die N-Acyl-N-(oxazolidin-2-on-3-yl)-2,6-dialkyl-3-chloraniline der Formel I, indem man ein 2,6-Dialkyl-anilin der Formel II durch Reaktion mit Natrium- oder Kaliumnitrit diazotiert, das entstandene 2,6-Dialkylphenyldiazoniumsalz mit Zinn(II)-chlorid zum 2,6-Dialkylphenylhydrazin der Formel III reduziert, dieses durch Reaktion mit einem Halogenameisensäure-halogenäthyl ester der Formel IV bei einer Temperatur zwischen +20°C und +30°C in Toluol oder Xylol in eine Verbindung der Formel V überführt, dieses Zwischenprodukt in Toluol oder Xylol bei einer Temperatur zwischen +40°C und dem Siedepunkt der Reaktionsmischung mit einem Acylierungsmittel der Formel VI umsetzt, das erhaltene Produkt bei einer Temperatur zwischen 0°C und +100°C in Methanol oder Aethanol in Gegenwart von Natrium- oder Kaliumhydroxid cyclisiert; und das entstehende Zwischenprodukt der Formel VII durch Einwirkung von Chlor in Gegenwart von Ameisensäure mit einem Wassergehalt von bis zu 40 Gew.% in Gegenwart von 1,5-2,5 Gew.% Eisen(III)chlorid bezogen auf

eingesetztes Produkt der Formel VII mit Chlorgas bei einer Temperatur zwischen +20°C und +30°C in 3-Stellung einfach oder bei einer Temperatur zwischen +40°C und +70°C in 3- und in 5-Stellung zweifach chloriert.

Durch das erfindungsgemässe Verfahren wird es möglich, die N-Acyl-N-(oxazolidin-2-on-3-yl)-2,6-dialkyl-3-chloraniline der Formel I ausgehend von 2,6-Dialkylanilinen der Formel II in einfacher Weise und in wesentlich besserer Ausbeute herzustellen als mit den bekannten Verfahren. Gegenüber den in den GB-A-2 058 071 und der DE-OS 3 030 026 beschriebenen Verfahren, welche auf der Chlorierung von N-Acetyl-2,6-dialkylanilinen beruhten, entfallen zwei Reaktionsstufen, nähmlich die Einführung der Acetylgruppe vor der Chlorierung und ihre Abspaltung nach der Chlorierung. Ausserdem werden überraschenderweise bei der Chlorierung von N-Acyl-N-(oxazolidin-2-on-3-yl)-2,6-dialkylanilinen der Formel VII bessere Ausbeuten erreicht als bei der bekannten Chlorierung von 2,6-Dialkylacetaniliden. Diese Vorteile werden erst durch das erfindungsgemässe Konzept nutzbar, dessen wesentliches Merkmal darin besteht, dass die Einführung des Chloratoms in 3-Stellung des Phenylrestes in der letzten Stufe durchgeführt wird.

Das erfindungsgemässe Verfahren wird durch die folgenden Beispiele näher erläutert. Das Beispiel 2 kann wahlweise die Stufe e) des Beispiels 1 ersetzen, während das Beispiel 3 die Steuerung des erfindungsgemässen Verfahrens zu den Verbindungen der Formel I, worin $R^2$ für Chlor steht, erläutert.

Beispiel 1: N-Methoxyacetyl-N-(oxazolidin-2-on-3-yl)-2,6-dimethyl-3-chloranilin

a) 2,6-Dimethylphenylhydrazin-Hydrochlorid.

Eine Mischung von 250 ml 36%iger Salzsäure, 110 ml Wasser und 121 g 2,6-Dimethylanilin wird bei -5°C tropfenweise mit einer Lösung von 73,7 g Natriumnitrit in 110 ml Wasser versetzt. Zur entstandenen klaren, orangefarbenen Lösung lässt man bei einer Temperatur von 0°C eine Lösung von 506,5 g Zinn(II)chlorid-Dihydrat in 675 ml 18%iger Salzsäure zutropfen. Die Reaktionsmischung wird für 18 Stunden bei Raumtemperatur gerührt. Der kristalline Niederschlag wird abgetrennt und mit Diäthyläther gewaschen. Man erhält 240 g eines festen Zwischenprodukts, das man in 1 Liter Wasser dispergiert. Bei einer Temperatur von +15°C setzt man eine Lösung von 253 g Natriumhydroxid in 340 ml Wasser zu und extrahiert mehrmals mit Diäthyläther. Die organische Phase wird über Natriumsulfat getrocknet, filtriert und mit trockenem HCl-Gas behandelt bis kein Hydrochlorid mehr ausfällt. Das ausgefällte 2,6-Dimethylphenylhydrazin-Hydrochlorid wird abgetrennt und getrocknet. Man erhält so 114 g des Produkts, Smp. 228°C (Zers.).

b) N-(2,6-Dimethylphenyl)-N′-(2-bromäthoxycarbonyl)-hydrazin.

Zu einer Lösung von 55,6 g Triäthylamin und 42,9 g 2,6-Dimethylphenylhydrazin-Hydrochlorid in 300 ml Toluol lässt man bei Raumtemperatur und unter Rühren eine Lösung von 51,5 g Chlorameisensäure-2-bromäthylester in 50 ml Toluol tropfen. Nachdem die Reaktionsmischung noch für eine weitere Stunde bei Raumtemperatur gerührt worden ist, wird sie mit 2N-wässriger Natriumhydroxidlösung gewaschen, über Natriumsulfat getrocknet, filtriert und eingedampft. Man erhält als Rückstand 68 g N-(2,6-Dimethylphenyl)-N′-(2-bromäthoxycarbonyl)-hydrazin in Form eines Oeles, das ohne weitere Reinigung im anchliessenden Reaktionsschritt eingesetzt werden kann.

c) N-Methoxyacetyl-N-(2,6-dimethylphenyl)-N′-(2-bromäthoxycarbonyl)-hydrazin.

Zu einer Lösung von 40,2 g N-(2,6-Dimethylphenyl)-N′-(2-bromäthoxycarbonyl)-hydrazin in 150 ml wasserfreiem Toluol lässt man bei Raumtemperatur eine Lösung von 18,5 g Methoxyacetylchlorid in 50 ml Toluol zutropfen. Das Gemisch wird unter Einleitung von Stickstoffgas für 16 Stunden zum Rückfluss erhitzt. Durch Abdampfen des Lösungsmittels erhält man 25,5 g N-Methoxyacetyl-N-(2,6-dimethylphenyl)-N′-(2-bromäthoxycarbonyl)-hydrazin in Form eines Oeles, das ohne Reinigung im folgenden Reaktionsschritt verwendet werden kann.

d) N-Methoxyacetyl-N-(oxazolidin-2-on-3-yl)-2,6-dimethylanilin.

24,8 g N-Methoxyacetyl-N-(2,6-dimethylphenyl)-N′-(2-bromäthoxycarbonyl)-hydrazin und 3,9 g Kaliumhydroxid werden bei Raumtemperatur in 300 ml absolutem Aethanol für 40 Stunden gerührt. Die Reaktionsmischung wird filtriert und eingeengt. Der Rückstand wird in Methylenchlorid aufgenommen, mit Wasser gewaschen, über Natriumsulfat getrocknet und eingedampft. Das zurückbleibende Oel wird durch Behandeln mit Petroläther zur Kristallisation gebracht. Durch Umkristallisieren aus Diäthyläther erhält man 11,5 g N-Methoxyacetyl-N-(oxazolidin-2-on-3-yl)-2,6-dimethylanilin mit einem Schmelzpunkt von 95-101°C.

e) Einer Lösung von 11,7 g N-Methoxyacetyl-N-(oxazolidin-2-on-3-yl)-2,6-dimethylanilin in 100 ml Chloroform werden 0,5 g wasserfreies Eisen-III-chlorid zugesetzt. Innerhalb von 8 Stunden leitet man bei einer Temperatur zwischen +20°C und +25°C in diese Mischung 10 g Chlorgas ein. Das Reaktionsgemisch wird mit Wasser gewaschen, über Natriumsulfat getrocknet, filtriert und eingedampft. Das ölige Rohprodukt wird chromatographisch an Kieselgel mit einem Aethyl acetat/Aether/Hexan-Gemisch (3:2:1) gereinigt. Man erhält so 5,5 g (42 % d. Th.) N-Methoxyacetyl-N-(oxazolidin-2-on-3-yl)-2,6-dimethyl-3-chloranilin in Form eines viskosen Oeles, $n_D^{50}$ = 1.5380.

Beispiel 2: N-Methoxyacetyl-N-(oxazolidin-2-on-3-yl)-2,6-dimethyl-3-chloranilin

11,7 g N-Methoxyacetyl-N-(oxazolidin-2-on-3-yl)-2,6-dimethylanilin und 0,5 g Eisen-III-chlorid werden in 100 ml 85%iger Ameisensäure gelöst. Bei einer Temperatur zwischen +20°C und +25°C werden in diese Lösung innerhalb von 4 Stunden 2,5 g Chlorgas eingeleitet. Das Reaktionsgemisch wird in einer Mischung von 2 l

Wasser und 200 ml Chlorform aufgenommen. Die organische Phase wird mit gesättigter Natriumhydrogencarbonatlösung und Wasser gewaschen, über Natriumsulfat getrocknet und eingedampft. Nach der chromatographischen Reinigung am Kieselgel mit einem Aethylacetat/Aether/Hexan-Gemisch (3:1:1) erhält man 11,8 g (90 % d. Th.) N-Methoxyacetyl-N-(oxazolidin-2-on-3-yl)-2,6-dimethyl-3-chloranilin als viskoses Oel, $n_D^{50} = 1.5380$.

Beispiel 3: N-Methoxyacetyl-N-(oxazolidin-2-on-3-yl)-2,6-dimethyl-3,5-dichloranilin

11,3 g N-Methoxyacetyl-N-(oxazolidin-2-on-3-yl)-2,6-dimethylanilin werden in 80 ml 85%iger Ameisensäure gelöst. Diese Lösusng wird mit 0,4 g Eisen-III-chlorid versetzt und auf eine Temperatur zwischen +50°C und +60°C erhitzt. Bei dieser Temperatur leitet man innerhalb von 5 Stunden 9 g Chlorgas in die Reaktionsmischung ein. Nach dem Abkühlen auf Raumtemperatur wird das Reaktionsgemisch in 2 l Eiswasser eingegossen. Das ausgefallene Produkt wird abgetrennt, mit Wasser gewaschen und im Vakuum bei +60°C getrocknet. Man erhält so 13,1 g (94 % d. Th.) N-Methoxyacetyl-N-(oxazolidin-2-on-3-yl)-2,6-dimethyl-3,5-dichloranilin, Smp. 171-175°C.

In analoger Weise erhält man die in der angeschlossenen Tabelle 1 aufgelisteten Verbindungen der Formel I.

Tabelle 1:

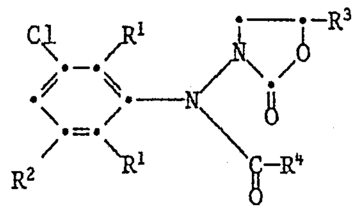

| Verb. Nr. | $R^1$ | $R^2$ | $R^3$ | $R^4$ | Physik. Daten |
|---|---|---|---|---|---|
| 1.01 | $CH_3$ | H | H | $-CH_2OCH_3$ | $n_D^{50}$ 1,5380 |
| 1.02 | $CH_3$ | H | H | 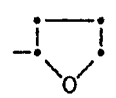 | |
| 1.03 | $CH_3$ | H | H | $-CH_2Cl$ | |
| 1.04 | $CH_3$ | Cl | H | $-CH_2OCH_3$ | Smp. 171-175° |
| 1.05 | $CH_3$ | H | $CH_3$ | $-CH_2OCH_3$ | |
| 1.06 | $CH_3$ | Cl | $CH_3$ | $-CH_2OCH_3$ | |
| 1.07 | $C_2H_5$ | H | H | $-CH_2OCH_3$ | |
| 1.08 | $C_2H_5$ | Cl | H | $-CH_2OCH_3$ | |
| 1.09 | $CH_3$ | Cl | H | 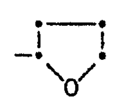 | |
| 1.10 | $CH_3$ | Cl | H | $-CH_2Cl$ | |
| 1.11 | $CH_3$ | H | $CH_3$ | $-CH_2Cl$ | |

**Patentansprüche**

1. Verfahren zur Herstellung von N-Acyl-N-(oxazolidin-2-on-3-yl)-2,6-dialkyl-3-chloranilinen der Formel I

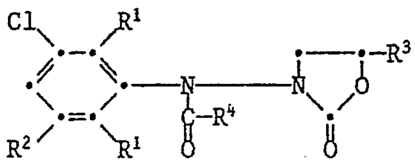

(I)

worin
$R^1$ Methyl oder Aethyl,
$R^2$ Wasserstoff oder Chlor,
$R^3$ Wasserstoff oder Methyl und
$R^4$ $C_1$-$C_3$-Alkoxymethyl, Halogenmethyl oder Tetrahydrofuryl darstellt, dadurch gekennzeichnet, dass man ein 2,6-Dialkylanilin der Formel II

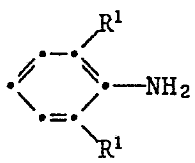

(II)

worin $R^1$ die unter Formel I gegebene Bedeutung hat, durch Reaktion mit einem Alkalnitrit diazotiert, das entstandene 2,6-Dialkylphenyldiazoniumsalz mit einem Reduktionsmittel zum 2,6-Dialkylphenylhydrazin der Formel III

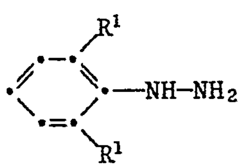

(III)

worin $R^1$ die unter Formel I gegebene Bedeutung hat, reduziert, dieses durch Reaktion mit einem Halogenameisensäure-2-halogenäthylester der Formel IV

$$Hal^1-CO-O-\underset{R^3}{CH}-CH_2-Hal^2 \qquad (IV)$$

worin $R^3$ die unter Formel I gegebene Bedeutung hat und $Hal^1$ und $Hal^2$ unabhängig voneinander für Chlor oder Brom stehen, in eine Verbindung der Formel V

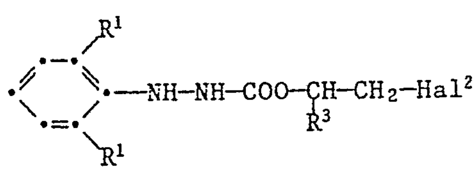

(V)

worin $R^1$ und $R^3$ die unter Formel I gegebene Bedeutung haben und $Hal^2$ für Chlor oder Brom steht, überführt, dieses Zwischenprodukt entweder zuerst mit einem Acylhalogenid der Formel VI

$Hal^3$-CO-$R^4$ (VI)

worin $R^4$ die unter Formel I gegebene Bedeutung hat und $Hal^3$ für Chlor oder Brom steht, umsetzt und anschliessend durch Behandlung mit einem säurebindenden Mittel intramolekular cyclisiert; oder zuerst durch Behandlung mit einem säurebindenden Mittel intramolekular cyclisiert und anschliessend mit einem Acylhalogenid der Formel V umsetzt; und das entstehende Zwischenprodukt der Formel VII

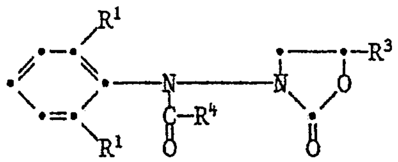

(VII)

worin $R^1$, $R^3$ und $R_4$ die unter Formel I gegebene Bedeutung haben, durch Einwirkung von Chlor in 3-Stellung einfach oder in 3- und 5-Stellung zweifach chloriert.

2. Verfahren nach Anspruch 1, dadurch gekennzeichnet, dass man als 2,6-Dialkylanilin der Formel II 2,6-Dimethylanilin oder 2,6-Diäthylanilin verwendet.

3. Verfahren nach Anspruch 2, dadurch gekennzeichnet, dass man als 2,6-Dialkylanilin der Formel II 2,6-Dimethylanilin verwendet.

4. Verfahren nach Anspruch 1, dadurch gekennzeichnet, dass man als Halogenameisensäure-halogenäthylester der Formel IV Chlorameisensäure-2-bromäthylester verwendet.

5. Verfahren nach Anspruch 1, dadurch gekennzeichnet, dass man als Acylierungsmittel der Formel VI Methoxyacetylchlorid, Chloracetylchlorid oder Tetrahydrofuran-2-carbonsäurechlorid verwendet.

6. Verfahren nach Anspruch 5, dadurch gekennzeichnet, dass man als Acylierungsmittel der Formel VI Methoxyacetylchlorid verwendet.

7. Verfahren nach Anspruch 1, dadurch gekennzeichnet, dass man die Umsetzung eines 2,6-Dialkylhydrazins der Formel III mit einem Halogenameisensäure-halogenäthylester der Formel IV in einem inerten Lösungsmittel durchführt.

8. Verfahren nach Anspruch 7, dadurch gekennzeichnet, dass man als inertes Lösungsmittel Benzol, Toluol, Xylol, Chlorbenzol, o-Dichlorbenzol, N,N-Dimethylformamid oder N,N-Dimethylacetamid verwendet.

9. Verfahren nach Anspruch 7, dadurch gekennzeichnet, dass man als inertes Lösungsmittel Toluol oder Xylol verwendet.

10. Verfahren nach Anspruch 1, dadurch gekennzeichnet, dass man die Umsetzung eines 2,6-Dialkylhydrazins der Formel III mit einem Halogenameisensäure-halogenäthylester der Formel IV bei einer Temperatur zwischen 0° C und +30° C durchführt.

11. Verfahren nach Anspruch 1, dadurch gekennzeichnet, dass man die Umsetzung eines 2,6-Dialkylhydrazins der Formel III mit einem Halogenameisensäure-halogenäthylester der Formel IV in Toluol oder Xylol als Lösungsmittel bei einer Temperatur zwischen +20° C und +30° C durchführt.

12. Verfahren nach Anspruch 1, dadurch gekennzeichnet, dass man das Zwischenprodukt der Formel V zuerst mit einem Acylhalogenid der Formel VI umsetzt und anschliessend durch Behandlung mit einem säurebindenden Mittel intramolekular cyclisiert.

13. Verfahren nach Anspruch 12, dadurch gekennzeichnet, dass man die Umsetzung eines Hydrazins der Formel V mit einem Acylierungsmittel der Formel VI in einem inerten Lösungsmittel durchführt.

14. Verfahren nach Anspruch 13, dadurch gekennzeichnet, dass man die Umsetzung eines Hydrazins der Formel V mit einem Acylierungsmittel der Formel VI in Hexan, Benzol, Toluol, Xylol, Chlorbenzol, Methylenchlorid, Chloroform, Kohlenstofftetrachlorid oder Aethylenchlorid als Lösungsmittel durchführt.

15. Verfahren nach Anspruch 13, dadurch gekennzeichnet, dass man die Umsetzung eines Hydrazins der Formel V mit einem Acylierungsmittel der Formel VI in Toluol oder Xylol als Lösungsmittel durchführt.

16. Verfahren nach Anspruch 13, dadurch gekennzeichnet, dass man die Reaktionstemperatur zwischen +40° C und der Siedetemperatur der Reaktionsmischung hält.

17. Verfahren nach Anspruch 13, dadurch gekennzeichnet, dass die Umsetzung des Hydrazins der Formel V mit einem Acylierungsmittel der Formel VI in Toluol oder Xylol bei einer Temperatur zwischen +40° C und der Siedetemperatur der Reaktionsmischung durchführt.

18. Verfahren nach Anspruch 12, dadurch gekennzeichnet, dass man die intramolekulare Cyclisierung in einem inerten Lösungsmittel in Gegenwart eines säurebindenden Mittels durchführt.

19. Verfahren nach Anspruch 18, dadurch gekennzeichnet, dass man bei der intramolekularen Cyclisierung als säurebindendes Mittel Alkali-oder Erdalkalimetallhydroxide, -carbonate oder -hydrogencarbonate, Triäthylamin oder Pyridin verwendet.

20. Verfahren nach Anspruch 19, dadurch gekennzeichnet, dass man als säurebindendes Mittel Natrium- oder Kaliumhydroxid verwendet.

21. Verfahren nach Anspruch 18, dadurch gekennzeichnet, dass die intramolekulare Cyclisierung in Methanol oder Aethanol durchgeführt wird.

22. Verfahren nach Anspruch 18, dadurch gekennzeichnet, dass man die intramolekulare Cyclisierung bei einer Temperatur zwischen 0° C und +100° C durchfürt.

23. Verfahren nach Anspruch 18, dadurch gekennzeichnet, dass man die intramolekulare Cyclisierung in Gegenwart von Natrium- oder Kaliumhydroxid bei einer Temperatur zwischen 0°C und +100°C in Methanol oder Aethanol ausführt.

24. Verfahren nach Anspruch 12, dadurch gekennzeichnet, dass man die Verbindung der Formel V in Toluol oder Xylol bei einer Temperatur zwischen +40°C und dem Siedepunkt der Reaktionsmischung mit einem Acylierungsmittel der Formel VI umsetzt und das erhaltene Produkt bei einer Temperatur zwischen 0°C und +100°C in Methanol oder Aethanol in Gegenwart von Natrium- oder Kaliumhydroxid cyclisiert.

25. Verfahren nach Anspruch 1, dadurch gekennzeichnet, dass man die Chlorierung eines Zwischenprodukts der Formel VII in einem inerten Lösungsmittel durchführt.

26. Verfahren nach Anspruch 25, dadurch gekennzeichnet, dass man Ameisensäure, Essigsäure, Chlorbenzol, Methylenchlorid, Chloroform, Kohlenstofftetrachlorid oder Aethylenchlorid als inertes Lösungsmittel verwendet.

27. Verfahren nach Anspruch 25, dadurch gekennzeichnet, dass man Ameisensäure oder Essigsäure mit einem Wassergehalt von bis zu 40 Gew.% als inertes Lösungsmittel verwendet.

28. Verfahren nach Anspruch 1, dadurch gekennzeichnet, dass man die Mono-Chlorierung eines N-Acyl-N-(oxazolidin-2-on-3-yl)-2,6-dialkylanilins der Formel VII bei Temperaturen von +20°C bis +30°C durchführt.

29. Verfahren nach Anspruch 28, dadurch gekennzeichnet, dass man die Mono-Chlorierung eines N-Acyl-N-(oxazolidin-2-on-3-yl)-2,6-dialkylanilins der Formel VII in Gegenwart von 1-5 Gew.% einer Lewis-Säure durchführt.

30. Verfahren nach Anspruch 29, dadurch gekennzeichnet, dass man als Lewis-Säure Aluminiumchlorid, Eisen(III)chlorid, Bortrifluorid oder Titantetrachlorid verwendet.

31. Verfahren nach Anspruch 29, dadurch gekennzeichnet, dass man als Lewis-Säure Eisen(III)chlorid verwendet.

32. Verfahren nach Anspruch 1, dadurch gekennzeichnet, dass man die zweifache Chlorierung eines N-Acyl-N-(oxazolidin-2-on-3-yl)-2,6-dialkylanilins der Formel VII bei Temperaturen von +40°C bis +70°C durchführt.

33. Verfahren nach Anspruch 32, dadurch gekennzeichnet, dass man die zweifache Chlorierung eines N-Acyl-N-(oxazolidin-2-on-3-yl)-2,6-dialkylanilins der Formel VII in Gegenwart von 1-5 Gew.% einer Lewis-Säure durchführt.

34. Verfahren nach Anspruch 33, dadurch gekennzeichnet, dass man als Lewis-Säure Aluminiumchlorid, Eisen(III)chlorid, Bortrifluorid oder Titantetrachlorid verwendet.

35. Verfahren nach Anspruch 33, dadurch gekennzeichnet, dass man als Lewis-Säure Eisen(III)chlorid verwendet.

36. Verfahren nach Anspruch 1, dadurch gekennzeichnet, dass man die Mono-Chlorierung eines N-Acyl-N-(oxazolidin-2-on-3-yl)-2,6-dialkylanilins der Formel VII bei +20°C bis +30°C in Ameisensäure mit einem Wassergehalt von bis zu 40 Gew.% in Gegenwart von 1,5-2,5 Gew.% Eisen(III)chlorid bezogen auf eingesetztes N-Acyl-N-(oxazolidin-2-on-3-yl)-2,6-dialkylanilin der Formel VII durchführt.

37. Verfahren nach Anspruch 1, dadurch gekennzeichnet, dass man die doppelte Chlorierung eines N-Acyl-N-(oxazolidin-2-on-3-yl)-2,6-dialkylanilins der Formel VII bei +40°C bis +70°C in Ameisensäure mit einem Wassergehalt von bis zu 40 Gew.% in Gegenwart von 1,5-2,5 Gew.% Eisen(III)chlorid bezogen auf eingesetztes N-Acyl-N-(oxazolidin-2-on-3-yl)-2,6-dialkylanilin der Formel VII durchführt.

38. Verfahren zur Herstellung von N-Acyl-N-(oxazolidin-2-on-3-yl)-2,6-dialkyl-3-chloranilinen der Formel I, dadurch gekennzeichnet, dass man ein 2,6-Dialkyl-anilin der Formel II durch Reaktion mit Natrium- oder Kaliumnitrit diazotiert, das entstandene 2,6-Dialkylphenyldiazoniumsalz mit Zinn(II)chlorid zum 2,6-Dialakylphenylhydrazinf der Formel III reduziert, dieses durch Reaktion mit einem halogenameisensäurehalogenäthylester der Formel IV bei einer Temperatur zwischen +20°C und +30°C in Toluol oder Xylol in eine Verbindung der Formel V überführt, dieses Zwischenprodukt in Toluol oder Xylol bei einer Temperatur zwischen +40°C und dem Siedepunkt der Reaktionsmischung mit einem Acylierungsmittel der Formel VI umseötzt, das erhaltene Produkt bei einer Temperatur zwischen 0°C und +100°C in Methanol oder Aethanol in Gegenwart von Natrium- oder Kaliumhydroxid cyclisiert; und das entstehende Zwischenprodukt der Formel VII durch Einwirkung von Chlor in Gegenwart von Ameisensäure mit einem Wassergehalt von bis zu 40 Gew.% in Gegenwart von 1,5-2,5 Gew.% Eisen(III)chlorid bezogen auf eingesetztes Produkt der Formel VII mit Chlorgas bei einer Temperatur zwischen +20°C und +30°C in 3-Stellung einfach oder bei einer Temperatur zwischen +40°C und +70°C in 3- und in 5-Stellung zweifach chloriert.

Europäisches Patentamt

# EUROPÄISCHER RECHERCHENBERICHT

Nummer der Anmeldung

EP 88 81 0141

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 030 923 (CIBA-GEIGY)<br>* Seiten 3-5,13-15; Ansprüche 1,7 *<br>--- | 1 | C 07 D 263/26<br>C 07 C 109/04<br>C 07 C 133/02 |
| D,A | GB-A-2 058 071 (MONTEDISON S.p.A.)<br>* Seite 3; Ansprüche *<br>--- | 1 | |
| D,A | DE-A-3 030 026 (SANDOZ)<br>* Ansprüche *<br>----- | 1 | |
| | | | **RECHERCHIERTE SACHGEBIETE (Int. Cl.4)** |
| | | | C 07 D 263/00 |

Der vorliegende Recherchenbericht wurde für alle Patentansprüche erstellt

| Recherchenort | Abschlußdatum der Recherche | Prüfer |
|---|---|---|
| DEN HAAG | 16-06-1988 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument